# EUROPÄISCHE PATENTANMELDUNG

(11) **EP 0 957 350 A2**
(43) Veröffentlichungstag der Anmeldung: **17.11.1999**
(21) Anmeldenummer: 99105777.9
(22) Anmeldetag: 22.03.1999
(51) Int. Cl.: G01N 1/22, G01N 33/497

(54) **Vorrichtung zur Probennahme für gasförmige Analyte**

(30) Priorität: 11.05.1998 DE 19820954
(71) Anmelder: SIEMENS AKTIENGESELLSCHAFT, 80333 München (DE)
(72) Erfinder: Bergner, Anne, Dipl.-Des., 81677 München (DE); Abraham-Fuchs, Klaus, Dipl.-Phys., 91058 Erlangen (DE)

(57) **Zusammenfassung**

Vorrichtung zur Probennahme für gasförmige Analyte umfassend:
eine Gaseinlaß- (14,14A) und eine Gasauslaßöffnung (16, 16A), mehrere gasleitend miteinander und mit der Gaseinlaß- und der Gasauslaßöffnung verbundene Kammern (2,4,6,8,10), wobei mindestens eine der Kammern flexible Kammerwände (18) besitzt, mindestens einen Sensor (25), der gasleitend mit mindestens einer der Kammern (2,4,6,8,10) verbunden ist, und zwei starre Formteile (20,20A;22,22A), die mit den flexiblen Kammerwänden (18) verbunden sind, wobei mindestens eines der starren Formteile (20,20A) an seiner den flexiblen Kammerwänden zugewandten Seite konkav ausgebildet ist.

## Beschreibung

Die Erfindung betrifft eine Vorrichtung zur Probennahme für gasförmige Analyte, insbesondere zur Probennahme von flüchtigen chemischen Verbindungen aus Stoffwechselvorgängen von Lebewesen, wie z.B. aus dem Atemgas.

Zur Analyse von Gasgemischen sind schon eine Vielzahl von Sensoren erhältlich, die in Abhängigkeit eines bestimmten Bestandteils ein Signal abgeben. Dabei ist für das Analysenergebnis eine reproduzierbare und kontrollierbare Probennahme wesentlich. Dabei dürfen beispielsweise das Probenvolumen, der Druck, die Flußgeschwindigkeit des Gasgemisches und die Temperatur nur in engen Bereichen schwanken. Bei der Analyse der Atemluft kommt hinzu, daß für besondere Applikationen gezielt ein im zeitlichen Verlauf definierter Anteil der Atemluft als Probe entnommen werden muß. Dies ist beispielsweise das endexpiratorische Atemvolumen, das heißt die Atemluft, die nicht aus dem oberen Luftweg, wie Luftröhre und Bronchialsystem, sondern direkt aus der Lunge kommt. Bei anderen Analysen kann es gerade ausgeschlossen sein, daß dieser Teil der Atemluft erfaßt wird, um dann nur Atemluft aus dem Rachenraum zu analysieren.

Ein Beispiel für eine Vorrichtung zur Probennahme aus Gasgemischen stellt ein Atem-Alkoholtestgerät dar. Dabei bläst ein Probant durch ein Röhrchen oder einen Schlauch die Atemluft direkt über die Sensoren. Ggf. ist auf dem Flußweg noch ein Flow Controller eingebaut, der den Fluß auf eine Obergrenze beschränkt.

So ist in der DE-OS 28 00 638 ein Meßgerät zur Bestimmung des Alkoholgehalts in dem Atem einer Versuchsperson beschrieben, bei dem in einer luftdicht abgeschlossenen Kammer, die einen Lufteintrittskanal zum Einblasen der Atemluft und einen Luftaustrittskanal mit einem Rückschlagventil aufweist, ein Gasdetektor angeordnet ist. Der elektrische Widerstand des Gasdetektors wird von dem Alkoholdampf beeinflußt. Der Verlauf des Lufteintrittskanals und die Lage seiner Ausmündung in die Kammer sind derart gewählt, daß die eingeblasene Luft nicht unmittelbar auf den Gasdetektor auftrifft. Zusätzlich besitzt die Kammer einen zweiten Eintrittskanal, der als Spülkanal dient und an dem ein Balg angeschlossen ist. Mittels des Balgs kann die Kammer nach Ablauf einer Messung mit reiner Luft ausgespült werden.

In der DE-AS 29 12 391 ist ein Gerät zur Lungenfunktionsanalyse beschrieben, das eine in einem Atembeutel angeordnete Meßsonde umfaßt. Ein von der Meßsonde abgenommenes gasdichteabhängiges Meßsignal entsprechend einer vorliegenden Testgaskonzentration wird unmittelbar als Lungenfunktionsmeßgröße dargestellt.

Die US-PS 4,723,435 beschreibt ein Atemsimulationsgerät. Das Gerät kann jeden Bereich der Atemleistung wiedergeben, indem atmosphärische Luft mit Kohlenstoffdioxid und Stickstoff gemischt und gepumpt wird. Das Gerät umfaßt Blase zum vorübergehenden Zwischenspeichern des Gasgemisches unter atmosphärischem Druck.

Klinische Tests der Atemluft werden häufig mittels der Gaschromatographie oder Massenspektrometrie durchgeführt. Dabei bläst der Probant häufig einen elastischen oder auch unelastischen Normbeutel aus Plastik bzw. Gummi auf, der anschließend in das Gerät gebracht wird, um die Probe dort automatisch aus diesem Volumen abzusaugen. Diese Methode sorgt für ein weitgehend konstantes Gasvolumen unter grob kontrollierten Druckverhältnissen, ist jedoch umständlich in der Handhabung.

Der Erfindung liegt nun die Aufgabe zugrunde, eine Vorrichtung zur Probennahme für gasförmige Analyte anzugeben, mit der unter gut reproduzierbaren Bedingungen eine Probennahme auch durch nicht für diese Aufgabe vorgebildete Laien, wie z.B. ein Patient, durchgeführt werden kann.

Die Aufgabe wird durch eine Vorrichtung gelöst, umfassend eine Gaseinlaß- und eine Gasauslaßöffnung, mehrere miteinander und mit der Gaseinlaß- und der Gasauslaßöffnung gasleitend verbundene Kammern, wobei mindestens eine der Kammern flexible Kammerwände besitzt, mit mindestens einem Sensor, der gasleitend mit mindestens einer der Kammern verbunden ist, und zwei starre Formteile, die mit den flexiblen Kammerwänden verbunden sind, wobei mindestens eines der starren Formteile an seiner den flexiblen Kammerwänden zugewandten Seite konkav ausgebildet ist. Durch die Anordnung der Kammer mit den flexiblen Seitenwänden zwischen den zwei starren Formteilen ist eine gute Handhabung und ein definiertes, nicht flexibles Teilvolumen zwischen den Schalen gegeben, nachdem die zwei starren Formteile zusammengedrückt oder geschlossen worden sind.

Bei einer vorteilhaften Ausgestaltung sind mindestens zwei Kammern in einer Strömungsrichtung hintereinander angeordnet. Dabei können bei zeitlich variabler Zusammensetzung des Gasgemisches verschiedene charakteristische Proben separiert werden. Im Beispiel der Atemluftanalyse können die Atemluft aus dem oberen Luftweg von der Atemluft aus der Lunge separiert werden.

Bei einer weiteren vorteilhaften Ausgestaltung sind zumindest einige der Kammern über Ventile miteinander verbunden. Damit können in Volumen, Druck und zeitlicher Abfolge definierte Teilvolumina des zu analysierenden Gasgemisches in verschiedenen Kammern gespeichert werden.

Bei einer weiteren vorteilhaften Ausgestaltung ist die Gasauslaßöffnung mit einem Überdruckventil verschlossen, um einen definierten Druck in den Kammern entstehen zu lassen.

Ein Eindringen der Umgebungsluft kann gemäß einer weiteren vorteilhaften Ausgestaltung verhindert werden, wenn die Gasauslaßöffnung mit einem Rückflußventil verschlossen ist.

Bei einer weiteren vorteilhaften Ausgestaltung ist in mindestens einer der Kammern ein Aktor angeordnet, der für eine konstante Bedingung des zu analysierenden Gasvolumens sorgt.

Optional können weitere Sensoren und/oder Aktoren vorgesehen sein, um Variationen in den Probenbedingungen bei der Auswertung berücksichtigen zu können. Dies sind: Gasflußmesser, Druckmesser, Überdruckventile, Mass Flow Controller (zur Einstellung eines konstanten Massenflusses), Rückflußventile, Temperatursensoren, Luftfeuchtesensoren.

Die Erfindung wird im folgenden anhand von drei Figuren erläutert. Es zeigen:
- Figur 1: in einer schematischen Seitenansicht eine erste Vorrichtung zur Probennahme mit mehreren Kammern,
- Figur 2: in einer Schnittdarstellung schematisch eine zweite Vorrichtung mit drei Kammern in einer ersten Arbeitsposition und
- Figur 3: in einer Schnittdarstellung die Vorrichtung nach Figur 2 in einer zweiten Arbeitsposition.

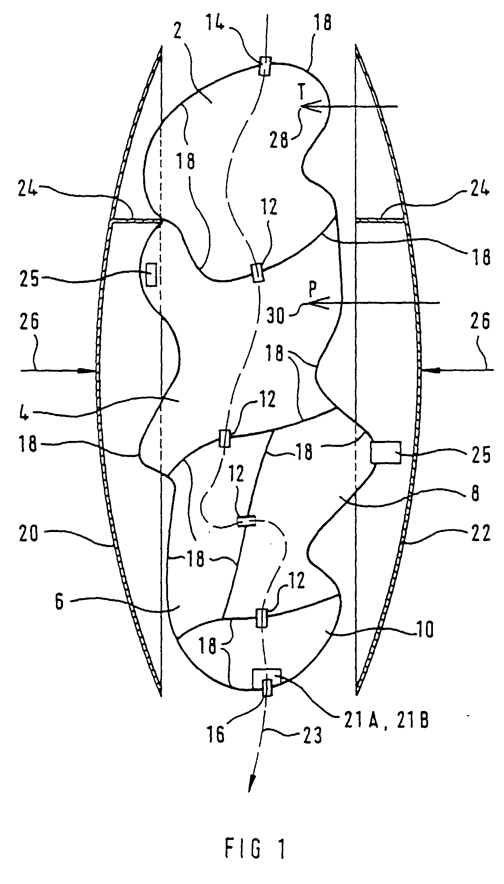

Die in Figur 1 in einer Seitenansicht gezeigte Vorrichtung zur Probennahme aus Gasgemischen umfaßt fünf aneinander hängende Kammern 2, 4, 6, 8, 10. Benachbarte Kammern sind über Leitungsverbindungen 12 verbunden. Die Kammer 2 umfaßt noch eine Gaseinlaßöffnung 14, die Kammer 10 eine Gasauslaßöffnung 16. Die Kammern 2, 4, 6, 8 und 10 besitzen flexible Kammerwände 16, wodurch ein Ausdehnen der einzelnen Kammern 2, 4, 6, 8, 10 entsprechend dem darin herrschenden Gasdruck ermöglicht wird. Die flexiblen Kammern 2, 4, 6, 8, 10 sind zwischen zwei festen kalottenförmigen Formteilen 20, 22 angeordnet. Die den Kammern 2, 4, 6, 8, 10 zugewandten Seiten der Formteile 20 und 22 sind konkav ausgebildet, wobei Stege 24 einzelne Bereiche abtrennen.

Das zu analysierende Gasgemisch wird der Vorrichtung zur Probennahme über die Gaseinlaßöffnung 14 zugeführt, wobei entsprechend einer eingezeichneten Strömungsrichtung 23 nach und nach die Kammern 2, 4, 6, 8, 10 gefüllt werden. Da die Wände 18 der Kammern 2, 4, 6, 8 flexibel ausgebildet sind, kann ein in weiten Grenzen schwankendes Gasvolumen von den einzelnen Kammern 2, 4, 6, 8 aufgenommen werden. Werden die beiden festen Formteile 20 und 22 geschlossen (symbolisiert durch Pfeile 26), so wird das Gasvolumen in den Kammern 2, 4, 6, 8 komprimiert oder über z.B. die Gasauslaßöffnung 16 ein entsprechender Anteil wieder entfernt. Ein an der Gasauslaßöffnung 16 angeordnetes Überdruckventil 21A begrenzt den Innendruck und ein dort angeordnetes Rückflußventil 21B verhindert das Eindringen von Umgebungsluft. Damit steht für die Gasanalyse in den einzelnen Kammern 2, 4, 6, 8, 10 jeweils ein definiertes Gasvolumen zur Verfügung. Beispielhaft ist in der Kammer 4 ein Sensor 25 angeordnet zur Messung eines Bestandteils des zu analysierenden Gasgemisches. In die Kammer 8 ragt nur eine sensitive Oberfläche eines weiteren Sensors 25, die übrigen Teile des Sensors 25 befinden sich außerhalb der Kammer 8. In den Kammern angeordnete Aktoren oder auch Sensoren, z.B. ein Temperatursensor/-aktor 28 in Kammer 2 und ein Drucksensor/-aktor 30 in Kammer 4 erlauben es, weitere Parameter des zu analysierenden Gases konstant zu halten.

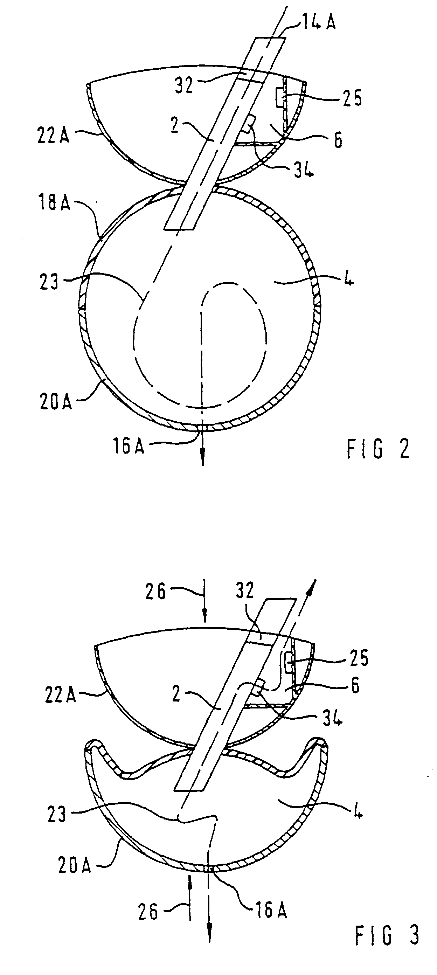

Die in Figur 2 dargestellte Vorrichtung zur Probennahme ist Teil eines Meßgeräts für natürliche Familienplanung, bei dem der Partialdruck des Kohlendioxids in der endexpiratorischen Atemluft analysiert wird. Diese Methode wurde von Professor Dr. Ludwig Wildt entwickelt und mit ihr läßt sich der Ovulationszeitpunkt bei Frauen ermitteln. Die Vorrichtung umfaßt zwei Kammern 2, 4, die hintereinander leitend verbunden sind. Eine dritte Kammer 6 ist neben der Kammer 2 angeordnet. Die Kammer 4 ist begrenzt von einem festen kalottenförmigen Formteil 20A und weiter von einem flexiblen Kammerwandteil 18A. Die Gaseinlaßöffnung 14A ist als Mundstück ausgebildet. Die Gasauslaßöffnung 16A befindet sich in der Mitte des festen Formteils 20A. In der Kammer 6 befindet sich ein Sensor 25 zur Messung des Kohlendioxid-Partialdrucks (pCO₂-Sensor). Das zweite feste Formteil 22A ist ebenfalls als Kugelkalotte ausgebildet, jedoch im Gegensatz zur in Figur 1 dargestellten Ausführung mit der konvexen Seite dem flexiblen Kammerwandteil zugewandt. Das Formteil 22A ist von den äußeren Abmessungen so dimensioniert, daß es die flexible Kammerwand 18A in den Innenraum des ersten Formteils 20A drücken kann.

In der Anwendung wird die komplette Atemluft über die Gaseinlaßöffnung 14A in das Gerät geblasen. Überflüssige Atemluft kann durch die Gasauslaßöffnung 16A entweichen. Nach dem vollständigen Ausatmen befindet sich in der Kammer 4 und insbesondere auch in der Kammer 2 das zu analysierende endexpiratorische Atemvolumen.

Durch Schließen der beiden Formteile 20A und 22A, was z.B. durch Handtbetätigung erfolgt, wird ein hinter der Gaseinlaßöffnung 14A angeordnetes Klappenventil 32 geschlossen und gleichzeitig ein die Kammern 2 und 6 verbindendes Klappenventil 34 geöffnet. Beim Schließen kann zusätzlich mit z.B. einem Finger die Gasauslaßöffnung 16A verschlossen werden. Damit strömt beim Zusammenpressen der beiden Formteile 20A und 22A die Atemluft in die Meßzelle 6. Die Meßzelle 6 ist so dimensioniert, daß eine zuverlässige Messung des gewünschten Gasanteils - hier pCO₂ - erfolgen kann. Eine hier nicht weiter dargestellte Auswerteelektronik und eine damit verbundene Anzeigeeinrichtung auf der Oberseite des festen Formteils 22A zeigt die gemessenen Werte an.

## Patentansprüche

1. Vorrichtung zur Probennahme für gasförmige Analyte mit
- einer Gaseinlaß- (14,14A) und einer Gasauslaßöffnung (16, 16A),
- mehreren miteinander und mit der Gaseinlaß- und der Gasauslaßöffnung gasleitend verbundenen Kammern (2,4,5,8,10), wobei mindestens eine der Kammern flexible Kammerwände (18) besitzt,
- mit mindestens einem Sensor (25), der gasleitend mit mindestens einer der Kammern (4,6) verbunden ist, und mit
- zwei starren Formteilen (20,20A;22,22A), die mit den flexiblen Kammerwänden (18) verbunden sind, wobei mindestens eines der starren Formteile (20,20A) an seiner den flexiblen Kammerwänden (18) zugewandten Seite konkav ausgebildet ist.

2. Vorrichtung nach Anspruch 1, **dadurch gekennzeichnet,** daß die beiden starren Formteile (20,20A;22,22A) auf den den flexiblen Kammerwänden (18) zugewandten Seiten Stege (24) aufweisen, welche Stege (24) beim Schließen der Formteile (20,20A;22,22A) Kammern (2,4) voneinander gasdicht abschließen.

3. Vorrichtung nach einem der Ansprüche 1 oder 2, **dadurch gekennzeichnet,** daß mindestens zwei Kammern (2,4,6,8,10) in einer Strömungsrichtung (23) hintereinander angeordnet sind.

4. Vorrichtung nach einem der Ansprüche 1 bis 3, **dadurch gekennzeichnet,** daß zumindest einige der Kammern über Ventile (32,34) miteinander verbunden sind.

5. Vorrichtung nach einem der Ansprüche 1 bis 4, **dadurch gekennzeichnet,** daß die Gasauslaßöffnung (16,16A) in der Kammer mit den flexiblen Kammerwänden (18) angeordnet ist.

6. Vorrichtung nach einem der Ansprüche 1 bis 5, **dadurch gekennzeichnet,** daß die Gasauslaßöffnung (16) mit einem Überdruckventil (21A) verschlossen ist.

7. Vorrichtung nach einem der Ansprüche 1 bis 6, **dadurch gekennzeichnet,** daß die Gasauslaßöffnung (16) mit einem Rückflußventil (21B) verschlossen ist.

8. Vorrichtung nach einem der Ansprüche 1 bis 7, **dadurch gekennzeichnet,** daß in mindestens einer der Kammern (2,4) ein Aktor (28,30) angeordnet ist.

9. Vorrichtung nach einem der Ansprüche 1 bis 8, **dadurch gekennzeichnet,** daß die Einlaßöffnung (14A) mit einem der starren Formteile (22A) verbunden ist.

10. Vorrichtung nach einem der Ansprüche 1 bis 9, **dadurch gekennzeichnet,** daß eines der Formteile (22A) mit der konvexen Seite der flexiblen Kammerwand (18) zugewandt ist.
